# EUROPEAN PATENT APPLICATION

(11) **EP 0 589 851 A1**
(43) Date of publication of application: **30.03.1994**
(21) Application number: 93830362.5
(22) Date of filing: 06.09.1993
(51) Int. Cl.: C12N 15/00, A01K 67/027, C12N 15/85, C12N 15/37

(54) **Transgenic non-human mammals with an oncogene under the control of an inducible liver-specific promoter**

(30) Priority: 07.09.1992 IT RM920651
(71) Applicant: ISTITUTO DI RICERCHE DI BIOLOGIA MOLECOLARE P. ANGELETTI S.P.A., 00040 Pomezia (IT)
(72) Inventor: Ciliberto, Gennaro, I-00124 Casalpalocco RM (IT)
(74) Representative: Di Cerbo, Mario

(57) **Abstract**

A transgenic non-human mammal, characterized in that all its germ cells and somatic cells contain a recombinant activated oncogene - under the control of an inducible liver specific promoter - introduced into said mammal, or an ancestor of said mammal, at an embryonic stage of its development. Extremely satisfactory results were obtained when the oncogenic sequence comprised the sequence coding for the big T-antigen of SV 40 virus and when the inducible liver specific promoter controlling the oncogene is the promoter of the human gene coding for C-reactive protein.

The figure represents the genetic map of a plasmid containing the above genic construct.

## Description

The present invention has as its subject a transgenic non-human mammal (preferably a rodent, for example a mouse), all of whose germ cells and somatic cells contain an activated oncogenic sequence - under the control of an inducible liver specific promoter - introduced into the animal, or an ancestor thereof, at an embryonic stage. (Activated oncogenic sequence is intended to mean an oncogene which, when incorporated into the genome of the animal, increases the probability of development of neoplasms, in particular malignant tumors).

As is known, the problem of explaining the phenomena leading to the appearance of tumors in mammals has been the object of serious research for many years. Today it is almost entirely certain that many stages are necessary to fully transform a cell and, thereby, to give rise to a tumor. Several research groups have adopted an approach based on the use of transgenic mice to investigate the requirements for a tumor to develop. To obtain transgenic mice, activated oncogenes were injected into fertilized eggs. This system has proved to be extremely useful to explain the relationship between overexpression of a given oncogene and the appearance of specific malignancies. More recently, mice have been generated which are deficient in the tumor suppressor p53. These mice showed a high susceptibility to the development of spontaneous tumors in various tissues. All these studies, however, have shown that an oncogene activation or tumor suppressor inactivation, taken singly, cannot cause the establishment of a tumour. On the contrary, secondary steps are necessary, such as, for example, the activation of cooperating oncogenes.

Although these studies have provided insights into the process of tumorigenesis, there are still several questions left open. It will only be possible to answer these if the expression of transgenic oncogenes can be more widely controlled, not only as regards tissue-specificity, but also as regards the duration of activation and the age of the animals.

The present invention provides, in the form of a transgenic non-human mammal, a living system for analysis of the sequence of events occurring during cancerogenesis of the liver.

In particular, the present invention relates to a transgenic non-human mammal, characterized in that all its germ cells and somatic cells contain a recombinant activated oncogene - under the control of an inducible liver specific promoter - introduced into said mammal, or an ancestor of the mammal, at an embryonic stage of development.

The oncogene used can be selected from the group comprising oncogenes having a sequence corresponding to endogenous coding sequences, or derived from oncogenic viruses.

The oncogenic sequence can be integrated in the chromosome of said mammal in a site different from the localization of said endogenous coding sequence.

In a preferred embodiment, the oncogenic sequence comprises the sequence coding for the big T-antigen of SV 40 virus.

In another preferred embodiment, the inducible liver-specific promoter is the human C-reactive protein gene promoter.

In the following an example will be given, in particular, of a case in which said transgenic non-human mammal is a rodent, and specifically a mouse. However, any species of transgenic animal according to the invention can be used. In certain circumstances, for example, it might be preferable to use a species, for example an ape, which is closer to man than the mouse from an evolutionary point of view.

The invention also extends to a genic construct comprising an activated oncogene under the control of an inducible liver-specific promoter, in particular that of the human C-reactive protein gene promoter.

A further object of the present invention is a modified plasmid, characterized in that it includes the above mentioned genic construct.

So far, a general description has been given of the subjects of the present invention. With the aid of the following examples, a detailed description of a specific embodiment of the invention will now be given, in order to more clearly understand the objects, characteristics, advantages and application thereof.

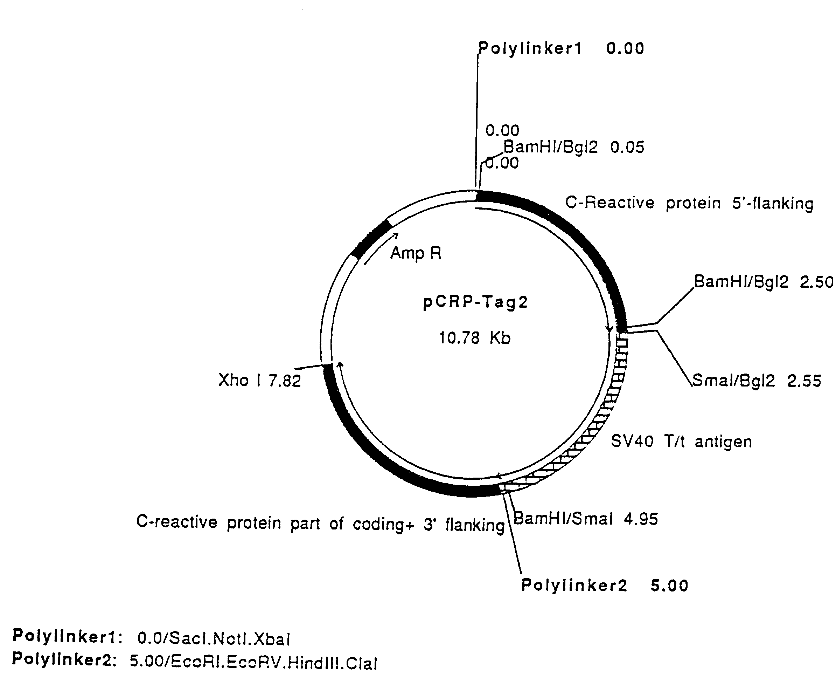

The sole figure enclosed herewith represents the genetic map of a plasmid containing a genic construct, according to the present invention, comprising the sequence coding for the big T-antigen of SV40 virus (indicated hereinafter as SV40 Tag or, more simply, Tag) under the control of an inducible liver-specific promoter of human C-reactive protein gene (hereinafter indicated as CRP promotor). Induction of said promotor takes place by injection of bacterial lyopolysaccharide (LPS).

### EXAMPLE 1

### Construction of CRP-Tag

A first plasmid was generated, BS-Tag, in which the BglI-BamHI DNA fragment from SV40 (Tooze J. (1980) DNA tumor viruses (Cold Spring Harbor, New York: Cold Spring Harbor Laboratory)) was cloned in the Sma site of vector Bluescript SK+ (Stratagene) after filling in both ends by DNA polymerase Klenow fragments (Boehringer, Mannheim). A plasmid carrying the insert in the desired orientation (with the AUG of the t/T ORFs immediately preceded by the BamHI site of the Bluescript polylinker) was selected by restriction mapping and DNA sequencing. BS-Tag was used for the construction of a second plasmid CRP-Tagl containing the CRP 5' flanking region cloned upstream to the SV40 T antigen coding region. The 2.5 kb BglII-BglII fragment carrying the CRP promoter and ending 16 bp downstream to the CRP cap site (Arcone et al, 1988) was cloned in the BamHI site of BS-Tag. The correct orientation was checked by restriction mapping and DNA sequencing. The CRP 3' fragment containing the second exon and 1.7 kb of 3' flanking sequences was derived from the 3' KpnI fragment of clone CRP-B5. After blunt-ending the KpnI sites the fragment was digested with BamHI (cuts 1.7 kb downstream of the coding sequence of CRP) and was subcloned into vector pCPXS. The fragment was reisolated as a ClaI - XhoI fragment and cloned into the pCRP-Tagl. From this new plasmid, pCRP-Tag2, the fragment for microinjection was gel purified after NotI and XhoI digestion.

### Deposit

The plasmid pCRP-Tag 2, the genetic map of which is shown in the sole figure enclosed, has been deposited in the National Collections of Industrial and Marine Bacteria Ltd. (NCIMB), Aberdeen, Scotland, UK, on 14 July 1992, under NCIMB accession No. 40516. Carrier cell: E. coli DH5oFl.

### EXAMPLE 2

### Generation of transgenic mice according to the invention, RNA analysis and bacterial lipopolysaccharide (LPS) induction

For the generation of transgenic mice according to the invention, eggs of F1 mice (C57BL/6 x SJL) were used for microinjection as basically described (Hogan, B.L.M., Costantini, F., Lacy, E. (1986) Manipulation of the mouse embryo: a laboratory manual. Cold Spring Harbor Laboratory, Cold Spring Harbor, New York). Using Southern analysis, ten mice resulting positive for the injected transgene were identified. All ten were bred and transmitted the CRP-Tag construct to offspring.

To assay the inducible and liver-specific expression of Tag, various animals of each of the ten CRP-Tag lines, derived independently, were sacrificed with and without injection of LPS. About ten different organs were isolated and used to extract RNA. Northern analysis revealed a highly varying expression profile of Tag between the individual lines, and made it possible to determine a line showing high expression levels, which was used for subsequent experiments. These experiments showed the close correlation between transgene induction and the appearance of hepatocellular carcinomas. It was found that induction of SV40 Tag for approximately 30 days is sufficient in all cases to start off development of liver tumors. This is the first case of a fully controlled oncogene expression and time-programmed tumor induction in a transgenic mouse.

During the experiments, it was noted that additional CRP 3' flanking sequences further downstream to those used in the construct according to the present invention, strongly contribute to ensure faithful regulation of CRP gene expression. It is therefore likely that the efficiency of the oncogene-bearing constructs can be improved by adding these sequences.

### EXAMPLE 3

### RNA analysis, LPS induction, Immunofluorescence analysis, histology and γ-GT assay

Isolation and analysis of RNA followed Standard protocols.

The injection of LPS for the 30, 60 and 90 days group were as follows: A stock solution of LPS (1µg/µl) was used and mice were injected every second day (in the morning between 9 and 10) with 100 µl of LPS (day 1-20), 150 µl of LPS (day 21-40), 200 µl of LPS (day 41-60) and 250 µl of LPS (day 61-90). The increase of LPS is necessary since the mice become resistant to lower concentrations over time. Controls injection with PBS followed the LPS protocol. At the end of the injection periods liver pieces were isolated by partial hepatectomy and stored for γ-glutamyltranspeptidase (γ-GT) activity assay and RNA analysis at -80°C or were fixed by 10% formaldehyde for histology.

For in situ analysis of Tag, livers were dissected, immediately frozen in liquid nitrogen and stored at -20°C. Sections of 6 um were performed on a cryostat at -20°C. Then, sections were dried for 10-30 minutes at room temperature and fixed in EtOH/HAc (95:5) for 5 minutes at 0°C. After blocking for 5 minutes in 10% FCS (in PBS), sections were stained for 30 minutes with a polyclonal anti Tag antibody generated in hamster. After several washes with PBS, sections were incubated for 30 minutes with an anti-hamster antibody which was Fluorescein conjugated (Dianova).

Histology was carried out as described by Rüther et al. (Rüther, U., Garber, C., Komitowski, D., Müller, R., Wagner, E.F. (1987) Deregulated c-fos expression interfers with normal bone development in transgenic mice. Nature 325, 412-416).

For the in situ assay of γ-GT activity the method described for frozen sections by Ogawa et al. was followed (Ogawa, K., Solt, D.B., Farber, E. (1980) Phenotypic diversity as an early property of putative preneoplastic hepatocyte populations in liver carcinogenesis. Cancer Res. 40, 725-733).

## Claims

1. A transgenic non-human mammal, characterized in that all of its germ cells and somatic cells contain a recombinant activated oncogene - under the control of an inducible liver specific promoter - introduced into said mammal, or an ancestor of the mammal, at an embryonic stage.

2. The transgenic non-human mammal as per claim 1, with a chromosome including an oncogene selected from the group comprising oncogenes, with a sequence corresponding to endogenous coding sequences, or derived from oncogenic viruses.

3. The transgenic non-human mammal as per claim 2, said oncogenic sequence being integrated in the chromosome of said mammal in a site different from the localization of said endogenous coding sequence.

4. The transgenic non-human mammal as per any one of the preceding claims, wherein the oncogenic sequence comprises the sequence coding for the big T-antigen of SV 40 virus.

5. The transgenic non-human mammal as per any one of the preceding claims, wherein the inducible liver-specific promoter is the human C-reactive protein gene promoter.

6. The transgenic non-human mammal as per claims 1 to 5, said mammal being a rodent.

7. The transgenic non-human mammal as per claim 6, said rodent being a mouse.

8. A genic construct, characterized in that it comprises an activated oncogene under the control of an inducible liver-specific promoter.

9. The genic construct according to claim 8, in which the oncogenic sequence comprises the sequence coding for the big T-antigen of SV40 virus and the inducible liver-specific promoter comprises the human C-reactive protein gene promoter.

10. A plasmid, characterized by the fact of including the genic construct of claim 9.

11. The plasmid according to claim 10, which includes a genic construct comprising the sequence coding for the big T-antigen of SV40 virus and the inducible liver-specific promoter of human C-reactive protein gene, said plasmid being deposited in the National Collections of Industrial and Marine Bacteria Ltd. with NCIMB access No. 40516.
